(19) Europäisches Patentamt
European Patent Office
Office européen des brevets

(11) Veröffentlichungsnummer: **0 355 601 B1**

# (12) EUROPÄISCHE PATENTSCHRIFT

(45) Veröffentlichungstag der Patentschrift: **01.03.95**

(51) Int. Cl.[6]: **C09B 33/24**, C09B 45/24, C07C 323/37, D06P 3/32, C07C 317/36

(21) Anmeldenummer: **89114812.4**

(22) Anmeldetag: **10.08.89**

## (54) Polyazofarbstoffe und deren Zwischenprodukte.

(30) Priorität: **12.08.88 DE 3827447**

(43) Veröffentlichungstag der Anmeldung:
**28.02.90 Patentblatt 90/09**

(45) Bekanntmachung des Hinweises auf die Patenterteilung:
**01.03.95 Patentblatt 95/09**

(84) Benannte Vertragsstaaten:
**AT CH DE ES FR GB IT LI**

(56) Entgegenhaltungen:
**EP-A- 0 101 664**
**US-A- 2 424 477**
**US-A- 2 438 754**

**J. Org. Chem., Band 21, S. 1382-1385, 1956**

(73) Patentinhaber: **BASF Aktiengesellschaft**
**Carl-Bosch-Strasse 38**
**D-67063 Ludwigshafen (DE)**

(72) Erfinder: **Hansen, Guenter, Dr.**
**Alwin-Mittasch-Platz 8**
**D-6700 Ludwigshafen (DE)**
Erfinder: **Zeidler, Georg**
**Mutterstadter Strasse 7**
**D-6701 Dannstadt-Schauernheim (DE)**
Erfinder: **Schaffer, Ortwin, Dr.**
**Luitpoldstrasse 119**
**D-6700 Ludwigshafen (DE)**

EP 0 355 601 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Polyazofarbstoffe der Formel I

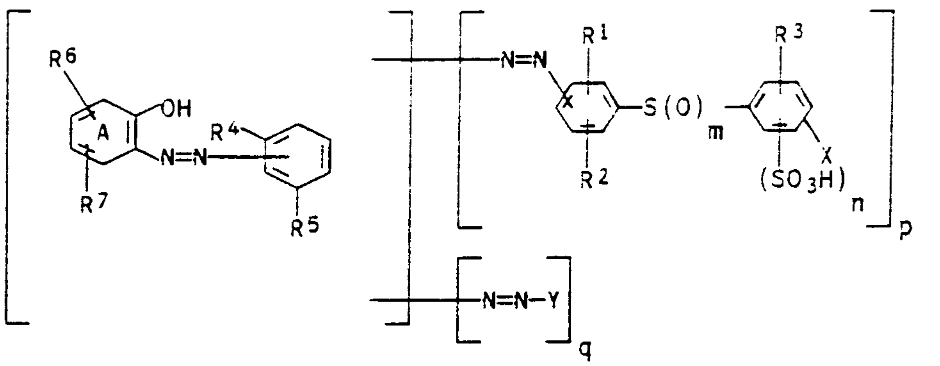

(I)

in freier Form oder als Kupfer-, Chrom-, Eisen-, Kobalt- oder Nickelkomplex, wobei

| | |
|---|---|
| m | 0, 1 oder 2 |
| n | 1, |
| p | 1 bis 2, |
| q | 0 bis 1, |
| X | Wasserstoff oder Nitro, |
| $R^1$ und $R^2$ | gleich oder verschieden sind und unabhängig voneinander jeweils Wasserstoff, $C_1$-$C_4$-Alkyl, $C_1$-$C_4$-Alkoxy, Halogen oder Hydroxysulfonyl, |
| $R^3$ | Wasserstoff, $C_1$-$C_4$-Alkoxy, Halogen, Carboxyl, Nitro oder $C_1$-$C_4$-Alkanoylamino, |
| $R^4$ und $R^5$ | gleich oder verschieden sind und unabhängig voneinander jeweils Hydroxy oder Amino, |
| $R^6$ | Wasserstoff, $C_1$-$C_4$-Alkyl, Halogen, Nitro, Hydroxysulfonyl, Sulfamoyl, $C_1$-$C_4$-Mono- oder -Dialkylsulfamoyl oder $C_1$-$C_4$-Alkylsulfonyl, |
| $R^7$ | Wasserstoff, Halogen, Nitro oder Hydroxysulfonyl und |
| Y | einen Rest der Formel |

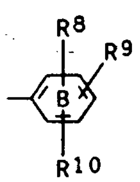

oder

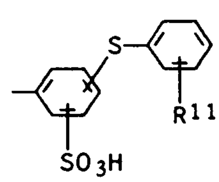

worin

| | |
|---|---|
| $R^8$ | für Wasserstoff, Hydroxy, Halogen, Nitro oder Hydroxysulfonyl, |
| $R^9$ | für Wasserstoff, Halogen, Nitro oder Hydroxysulfonyl, |
| $R^{10}$ | für Wasserstoff, $C_1$-$C_4$-Alkyl, $C_1$-$C_4$-Alkoxy oder Hydroxysulfonyl und |
| $R^{11}$ | für Wasserstoff, $C_1$-$C_4$-Alkyl, $C_1$-$C_4$-Alkoxy, Halogen oder Carboxyl stehen, bedeuten und die |

Ringe A und B jeweils durch einen gegebenenfalls durch Nitro oder Hydroxysulfonyl substituierten Benzoring anelliert sein können, sowie deren Zwischenprodukte.

In der CH-A-571 599, DE-A-2 024 047, DE-A-2 162 419 sowie EP-A-45 868 sind bereits Polyazofarbstoffe beschrieben, die denen der Formel I ähnlich sind. Anstelle der -$S(O)_m$-Brücke weisen sie jedoch eine Iminobrücke auf. Farbstoffe mit einer $SO_2$-Brücke werden in der US-A-2 438 754 beschrieben.

Aufgabe der vorliegenden Erfindung war es, neue Polyazofarbstoffe bereitzustellen, die leicht zugänglich sein und vorteilhafte anwendungstechnische Eigenschaften besitzen sollten.

Demgemäß wurden die Farbstoffe der obengenannten Formel I gefunden.

Alle in den erfindungsgemäßen Verbindungen auftretenden Alkylgruppen können sowohl geradkettig als auch verzweigt sein.

Reste $R^1$, $R^2$, $R^3$, $R^{10}$ und $R^{11}$ sind z.B. Methoxy, Ethoxy, Propoxy, Isopropoxy, Butoxy, Isobutoxy oder sec-Butoxy.

Reste $R^1$, $R^2$, $R^{10}$ und $R^{11}$ sind weiterhin, ebenso wie der Rest $R^6$, z.B. Methyl, Ethyl, Propyl, Isopropyl, Butyl, Isobutyl oder sec-Butyl.

Reste $R^1$, $R^2$, $R^3$, $R^6$ und $R^{11}$ sind weiterhin, ebenso wie die Reste $R^7$, $R^8$ und $R^9$, z.B. Fluor, Chlor oder Brom, wobei für den Fall, daß einer oder mehrere dieser Reste für Halogen steht, Chlor oder Brom bevorzugt ist.

$R^3$ bedeutet weiterhin z.B. Formylamino, Acetylamino, Propionylamino, Butyrylamino oder Isobutyrylamino.

$R^6$ bedeutet weiterhin z.B. Mono- oder Dimethylsulfamoyl, Mono- oder Diethylsulfamoyl, Mono- oder Dipropylsulfamoyl, Mono- oder Diisopropylsulfamoyl, Mono- oder Dibutylsulfamoyl, Methylsulfonyl, Ethylsulfonyl, Propylsulfonyl, Isopropylsulfonyl oder Butylsulfonyl.

Die Indices p und q können jeweils auch Bruchteile von ganzen Zahlen darstellen. Dies bedeutet, daß in diesem Fall Mischungen von Polyazofarbstoffen vorliegen.

Es versteht sich, daß für den Fall, daß in den Polyazofarbstoffen der Formel I eine oder mehrere Hydroxysulfonylgruppen enthalten sind, auch deren Salze von der Erfindung mit umfaßt werden sollen.

Als Salze kommen dabei Metall- oder Ammoniumsalze in Betracht. Metallsalze sind insbesondere die Lithium-, Natrium- oder Kaliumsalze. Unter Ammoniumsalzen im erfindungsgemäßen Sinne sind solche Salze zu verstehen, die entweder unsubstituierte oder substituierte Ammoniumkationen aufweisen. Substituierte Ammoniumkationen sind z.B. Monoalkyl-, Dialkyl-, Trialkyl-, Tetraalkyl- oder Benzyltrialkylammoniumkationen oder solche Kationen, die sich von stickstoffhaltigen fünf- oder sechsgliedrigen gesättigten Heterocyclen ableiten, wie Pyrrolidinium-, Piperidinium-, Morpholinium-, Piperazinium- oder N-Alkylpiperaziniumkationen oder deren N-monoalkyl- oder N,N-dialkylsubstituierte Produkte. Unter Alkyl ist dabei im allgemeinen geradkettiges oder verzweigtes $C_1$-$C_{20}$-Alkyl zu verstehen, das durch Hydroxylgruppen substituiert und/oder durch Sauerstoffatome unterbrochen sein kann.

Bevorzugt sind Polyazofarbstoffe der Formel Ia

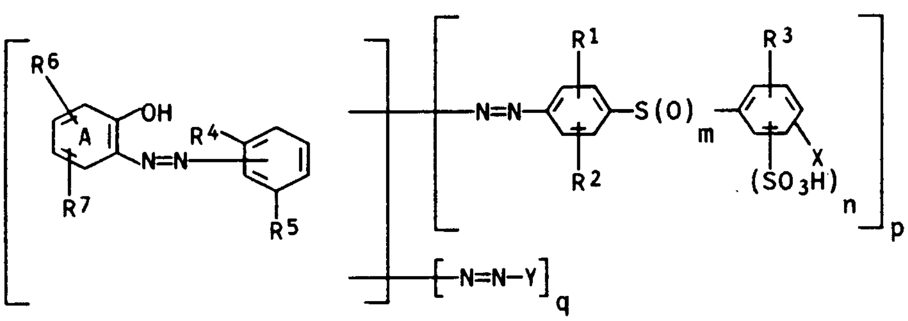 (Ia),

in der $R^3$ Wasserstoff bedeutet und $R^1$, $R^2$, $R^4$, $R^5$, $R^6$, $R^7$, X, Y, m, n, p, q sowie der Ring A jeweils die obengenannte Bedeutung besitzen.

Weiterhin bevorzugt sind Polyazofarbstoffe der Formel I, in der m 0 oder 2, q 0, X Nitro, $R^1$ und $R^2$ jeweils Wasserstoff und $R^3$ Wasserstoff oder Nitro bedeuten.

Insbesondere sind solche Polyazofarbstoffe der Formel I bevorzugt, in der m 0 bedeutet.

Wenn $R^3$ Wasserstoff und X Nitro bedeuten, sind insbesondere solche Farbstoffe der Formel I bevorzugt, in der die Nitrogruppe (X) in ortho-Position und die Hydroxysulfonylgruppe in para-Position zur $-S(O)_m$-Brücke oder die Nitrogruppe (X) in para-Position und Hydroxysulfonylgruppe in ortho-Position zur $-S(O)_m$-Brücke stehen.

Bevorzugt sind weiterhin die Kupfer-, Chrom-, Eisen- oder Kobaltkomplexe der Polyazofarbstoffe der Formel I, wobei die entsprechenden Eisen- oder Chromkomplexe besonders hervorzuheben sind.

Die erfindungsgemäßen Polyazofarbstoffe können nach an sich bekannten Methoden erhalten werden.

Beispielsweise kann man ein Aminophenol der Formel III

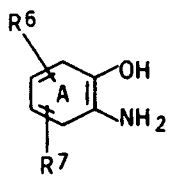

(III),

in der $R^6$ und $R^7$ und der Ring A jeweils die obengenannte Bedeutung besitzen, diazotieren und mit einer Kupplungskomponente der Formel IV

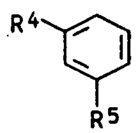

(IV),

in der $R^4$ und $R^5$ jeweils die obengenannte Bedeutung besitzen, kuppeln, wobei eine Monoazoverbindung der Formel V

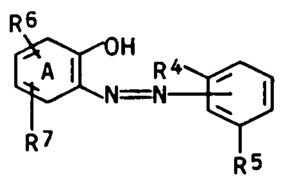

(V),

in der $R^4$, $R^5$, $R^6$, $R^7$ und der Ring A jeweils die obengenannte Bedeutung besitzen, resultiert.

Daran anschließend kann man dann ein Anilinderivat der Formel II

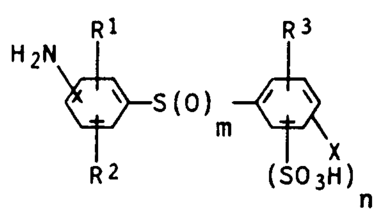

(II),

in der $R^1$, $R^2$, $R^3$, X, m und n jeweils die obengenannte Bedeutung besitzen, diazotieren und mit der oben beschriebenen Monoazoverbindung V kuppeln.

Gegebenenfalls kann man dann ein Amin der Formel VI

$Y\text{-}NH_2$ (VI),

in der Y die obengenannte Bedeutung besitzt, diazotieren und mit dem Diazofarbstoff der Formel I (mit q = 0) kuppeln.

Diese beispielhaft beschriebene Herstellungsweise braucht selbstverständlich nicht eingehalten zu werden, d.h. es ist auch möglich, die Kupplungsreihenfolge zu variieren. Beispielsweise kann man zuerst das Anilinderivat II diazotieren und mit der Kupplungskomponente IV kuppeln und das resultierende Reaktionsprodukt daran anschließend mit dem Aminophenol III, das vorher diazotiert wurde, kuppeln.

Die Metallisierung, d.h. die Herstellung der jeweiligen Kupfer-, Chrom-, Eisen-, Kobalt- oder Nickel komplexe der Polyazofarbstoffe I erfolgt ebenfalls nach an sich bekannten Methoden. Beispielsweise durch Behandlung des Polyazofarbstoffs mit den entsprechenden Metallsalzen, z.B. mit Eisen(III)chlorid, Eisen(II)-sulfat, Kupfersulfat oder Chrom(III)formiat in wäßriger Lösung bei einer Temperatur von 80 bis 105 °C und

einem pH-Wert von 4 bis 6.

Bei den Aminophenolen III und der Kupplungskomponente IV handelt es sich um an sich bekannte Verbindungen.

Ein weiterer Gegenstand der vorliegenden Erfindung sind Anilinderivate der Formel II

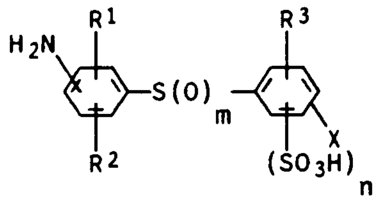

(II),

in der

| | |
|---|---|
| m | 0, |
| n | 1, |
| X | Wasserstoff oder Nitro, |
| $R^1$ und $R^2$ | gleich oder verschieden sind und unabhängig voneinander jeweils Wasserstoff, $C_1$-$C_4$-Alkyl, $C_1$-$C_4$-Alkoxy oder Hydroxysulfonyl, und |
| $R^3$ | Wasserstoff, $C_1$-$C_4$-Alkoxy, Carboxyl, Nitro oder $C_1$-$C_4$-Alkanoylamino bedeuten. |

Aus der EP-A-101 664, US-A-2 424 477 und J.Org.Chem., Band 21, Seiten 1382 bis 1385, 1956, sind ähnliche Verbindungen bekannt.

Für die beispielhafte Aufzählung der Reste $R^1$, $R^2$ und $R^3$ wird auf die oben bereits vorgenommenen Ausführungen verwiesen.

Die erfindungsgemäßen Anilinderivate der Formel II, die wertvolle Diazokomponenten für die Herstellung von Polyazofarbstoffen der Formel I oder auch von anderen Azofarbstoffen darstellen, können nach an sich bekannten Methoden erhalten werden.

Beispielsweise kann man eine Nitroverbindung der Formel VII

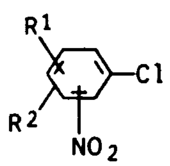

(VII),

in der $R^1$ und $R^2$ jeweils die obengenannte Bedeutung besitzen, mit einem Alkalisulfid zum Thiophenolderivat VIII

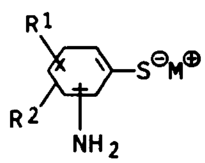

(VIII),

in der $M^{\oplus}$ ein Alkalimetallkation bedeutet und $R^1$ und $R^2$ jeweils die obengenannte Bedeutung besitzen, umsetzen.

Aus diesem Thiophenolderivat VIII entstehen durch Reaktion mit dem Chlorbenzolderivat der Formel IX

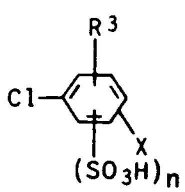

(IX),

in der $R^3$, X und n jeweils die obengenannte Bedeutung besitzt, die erfindungsgemäßen Anilinderivate der Formel II.

Durch Oxidation dieser Thioverbindungen, z.B. mit Wasserstoffperoxid, gelangt man zu entsprechenden Sulfoxiden oder Sulfonen.

Eine andere Möglichkeit für die Herstellung der Sulfone besteht beispielsweise darin, ein Sulfinat der Formel X

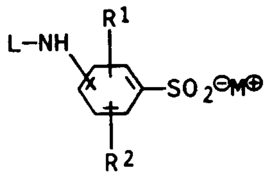

(X),

in der $R^1$ und $R^2$ jeweils die obengenannte Bedeutung besitzen und L für $C_1$-$C_4$-Alkanoyl und $M^{\oplus}$ für ein Alkalimetallkation stehen, mit dem Chlorbenzolderivat IX umzusetzen, wobei ein Sulfon der Formel XI

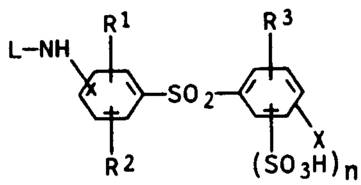

(XI)

resultiert, in der L, $R^1$, $R^2$, $R^3$, X und n jeweils die obengenannte Bedeutung besitzen. Durch hydrolytische Abspaltung der Schutzgruppe L gelangt man dann zur freien Aminoverbindung. In manchen Fällen erfolgt die hydrolytische Abspaltung der Schutzgruppe L auch bereits während der Sulfonbildung, d.h. das Sulfon XI wird nicht zwischenisoliert.

Die Sulfinate X sind z.B. durch Reduktion aus den entsprechenden Sulfonsäurechloriden zugänglich.

Bei den Komponenten der Formel VII, VIII, IX und X handelt es sich im allgemeinen um an sich bekannte Verbindungen.

Im folgenden seien beispielhaft einige Anilinderivate der Formel II sowie geeignete Aminophenolderivate der Formel III aufgeführt.

Anilinderivate der Formel II sind z.B.

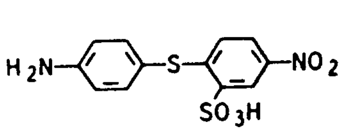 , 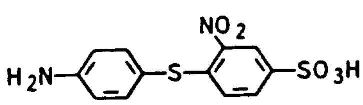 , 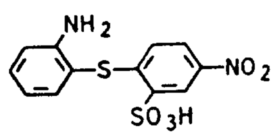 ,

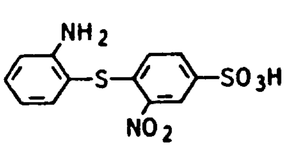 , 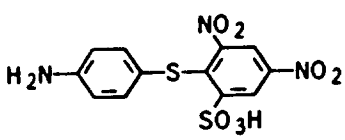 ,

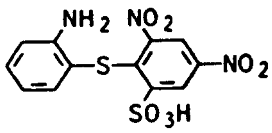 ,

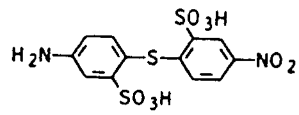,

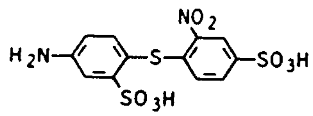, 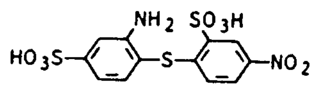 oder

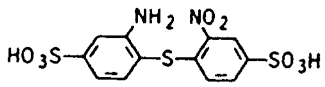.

Aminophenole der Formel III sind z.B.

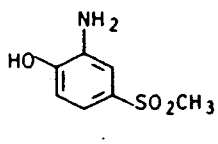, 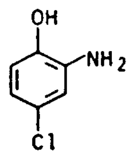, 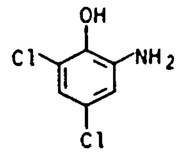, 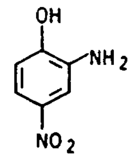,

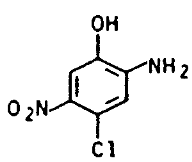, 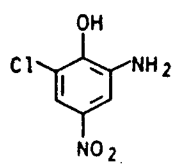, 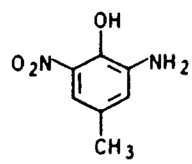, 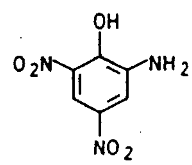,

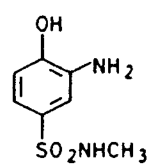, 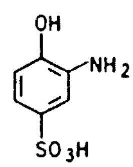, 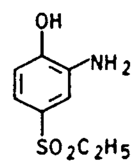, 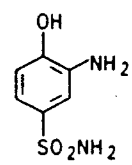,

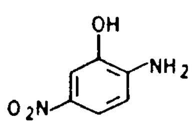, 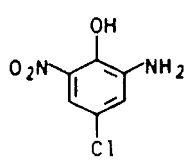, 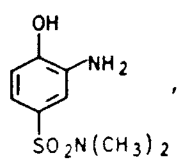, 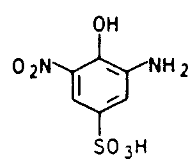,

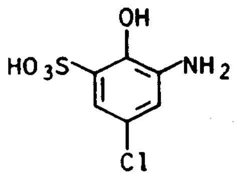 , 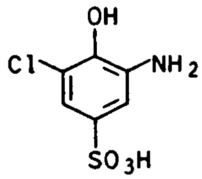 , 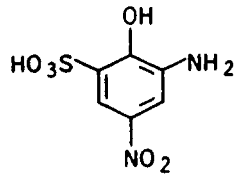 , 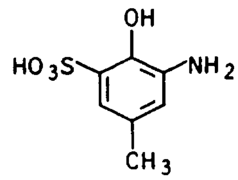

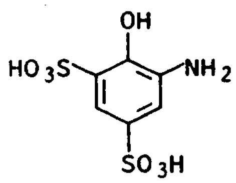 , 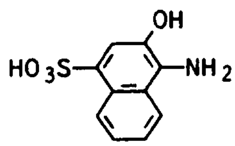 oder 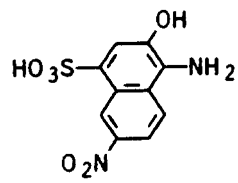 .

Die erfindungsgemäßen Polyazofarbstoffe eignen sich in vorteilhafter Weise zum Färben von Leder.

Die folgenden Beispiele sollen die Erfindung näher erläutern. Die dort genannten Prozente beziehen sich auf das Gewicht.

Beispiel 1

19,9 g 1-Amino-2-hydroxy-3,5-dinitrobenzol wurden in der üblichen Weise diazotiert und alkalisch auf 11 g Resorcin gekuppelt. Anschließend fügte man zu der Suspension bei pH 8-9 das Diazoniumsalz aus 32,6 g 4-Amino-4'-nitro-diphenylsulfid-2'-sulfonsäure hinzu. Nach beendeter Kupplung wurde das Reaktionsgemisch mit Essigsäure auf pH 5 eingestellt, mit 38 g wasserfreiem Eisen-III-chlorid versetzt und bei pH 4-5 ungefähr 3 Stunden auf 90 bis 95 °C erhitzt.

Nach erfolgter Metallisierung wurde der Farbstoff durch Zugabe von Natriumchlorid abgeschieden, isoliert und getrocknet. Der Eisenkomplex der Verbindung der Formel

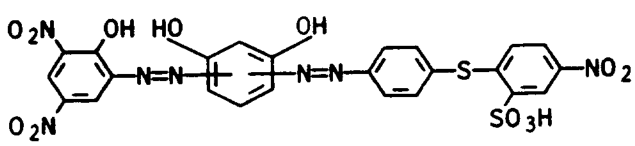

färbt Leder in licht- und naßechten gelbstichig braunen Tönen.

Die in den folgenden Tabellen aufgeführten Farbstoffe werden in analoger Weise erhalten.

Tabelle 1

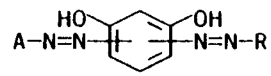

| Bsp. | A | R | Nuance der Färbung der Metallkomplexe auf Leder Cu | Cr | Co | Ni | Fe |
|---|---|---|---|---|---|---|---|
| 2 | 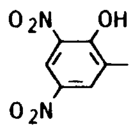 | 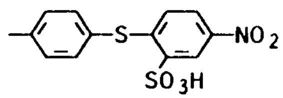 | rotbraun | | | rotbraun | |
| 3 | 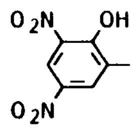 | 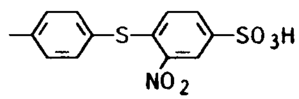 | | rotbraun | | | gelbbraun |
| 4 | 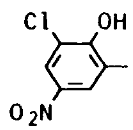 | 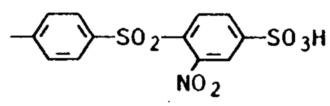 | braun | | | rotbraun | gelbbraun |
| 5 | 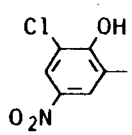 | 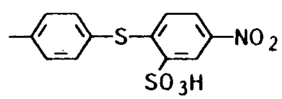 | rotbraun | | braun | | gelbbraun |
| 6 | 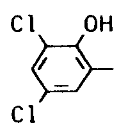 | 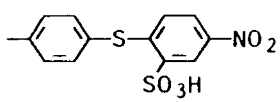 | | braun | | | gelbbraun |

Tabelle 1 (Forts.)

| Bsp. | A | R | Cu | Cr | Co | Ni | Fe |
|---|---|---|---|---|---|---|---|
| 7 | 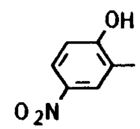 | 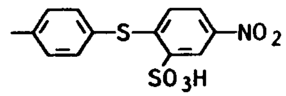 | | | rotbraun | | gelbbraun |
| 8 | 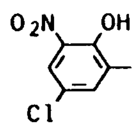 | 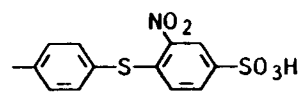 | | rotbraun | | | gelbbraun |
| 9 | 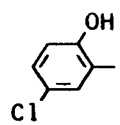 | 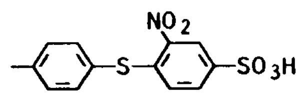 | rotbraun | | rotbraun | | |
| 10 | 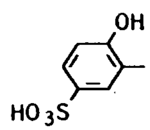 | 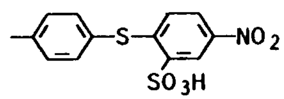 | rotbraun | | | | gelbbraun |
| 11 | 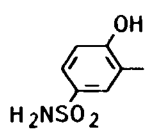 | 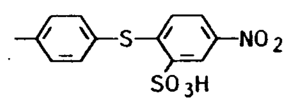 | | braun | | braun | gelbbraun |

Tabelle 1 (Forts.)

| Bsp. | A | R | Nuance der Färbung der Metallkomplexe auf Leder: Cu | Cr | Co | Ni | Fe |
|---|---|---|---|---|---|---|---|
| 12 | 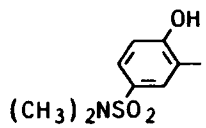 | 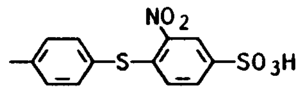 | | | rotbraun | | gelbbraun |
| 13 | 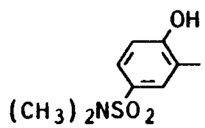 | 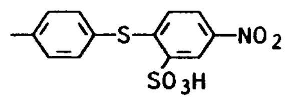 | | braun | | | gelbbraun |
| 14 | 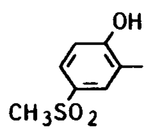 | 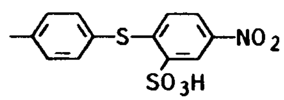 | rotbraun | | rotbraun | | gelbbraun |
| 15 | 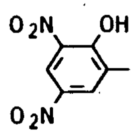 | 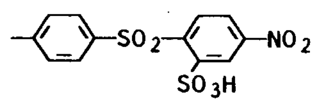 | | rotbraun | rotbraun | | gelbbraun |
| 16 | 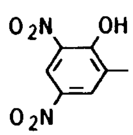 | 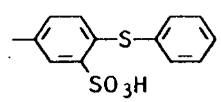 | rotbraun | rotbraun | | | gelbbraun |

Tabelle 1 (Forts.)

| Bsp. | A | R | Nuance der Färbung der Metallkomplexe auf Leder Cu | Cr | Co | Ni | Fe |
|---|---|---|---|---|---|---|---|
| 17 | 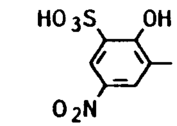 | 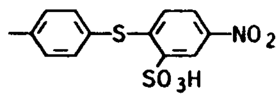 | rotbraun | | | | gelbbraun |
| 18 | 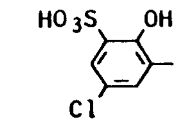 | 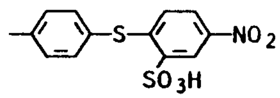 | | rotbraun | | | gelbbraun |
| 19 | 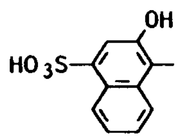 | 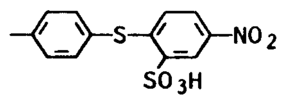 | | | | rotbraun | |
| 20 | 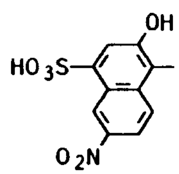 | 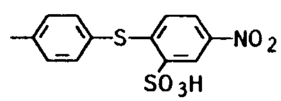 | rotbraun | | | | braun |
| 21 | 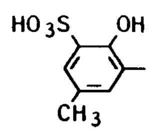 | 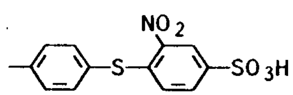 | | rotbraun | | | braun |

Tabelle 1 (Forts.)

| Bsp. | A | R | Nuance der Färbung der Metallkomplexe auf Leder: Cu | Cr | Co | Ni | Fe |
|---|---|---|---|---|---|---|---|
| 22 | 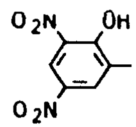 | 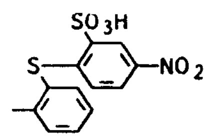 | rotbraun | | | | gelbbraun |
| 23 | 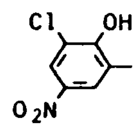 | 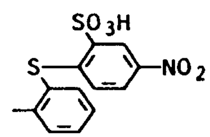 | | rotbraun | | | gelbbraun |

Tabelle 1 (Forts.)

| Bsp. | A | R | Nuance der Färbung der Metallkomplexe auf Leder | | | | |
|---|---|---|---|---|---|---|---|
| | | | Cu | Cr | Co | Ni | Fe |
| 24 | 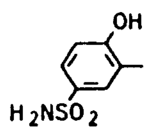 | 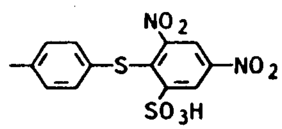 | | rotbraun | | | gelbbraun |
| 25 | 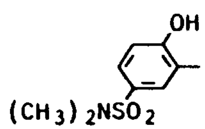 | 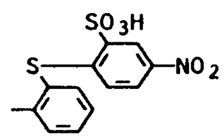 | | | rotbraun | | gelbbraun |

Tabelle 2

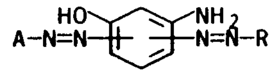

| Bsp. | A | R | Nuance der Färbung der Metallkomplexe auf Leder | | | | |
|---|---|---|---|---|---|---|---|
| | | | Cu | Cr | Co | Ni | Fe |
| 26 | 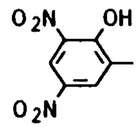 | 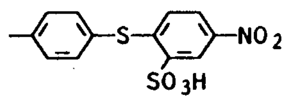 | rotbraun | | | | gelbbraun |
| 27 | 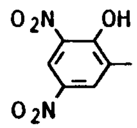 | 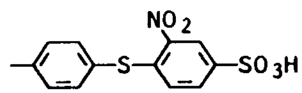 | | rotbraun | | | gelbbraun |
| 28 | 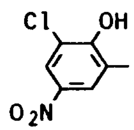 | 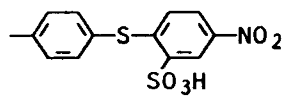 | | rotbraun | | rotbraun | |
| 29 | 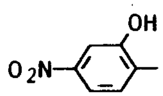 | 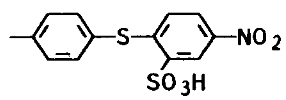 | rotbraun | | rotbraun | | braun |
| 30 | 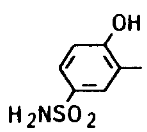 | 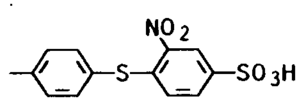 | | rotbraun | | | gelbbraun |

Tabelle 2 (Forts.)

| Bsp. | A | R | Nuance der Färbung der Metallkomplexe auf Leder: Cu | Cr | Co | Ni | Fe |
|---|---|---|---|---|---|---|---|
| 31 | 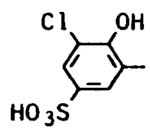 | 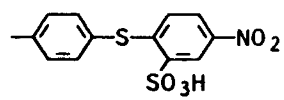 | | rotbraun | | | braun |
| 32 | 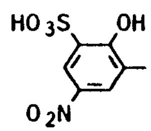 | 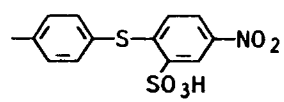 | rotbraun | | rotbraun | | |
| 33 | 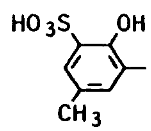 | 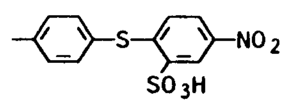 | | rotbraun | | | braun |

Tabelle 2 (Forts.)

| Bsp. | A | R | Nuance der Färbung der Metallkomplexe auf Leder: Cu | Cr | Co | Ni | Fe |
|---|---|---|---|---|---|---|---|
| 34 | 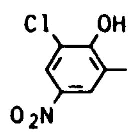 | 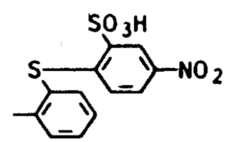 | rotbraun | | | rotbraun | |
| 35 | 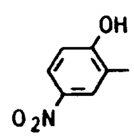 | 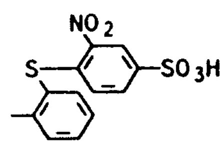 | | rotbraun | | | gelbbraun |

Tabelle 3

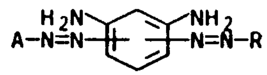

| Bsp. | A | R | Nuance der Färbung der Metallkomplexe auf Leder: Cu | Cr | Co | Ni | Fe |
|---|---|---|---|---|---|---|---|
| 36 | 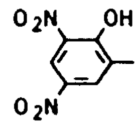 | 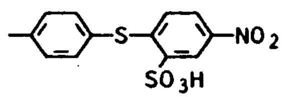 | rotbraun | | | | gelbbraun |
| 37 | 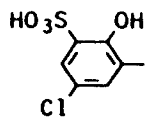 | 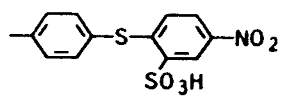 | | | | rotbraun | |
| 38 | 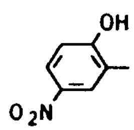 | 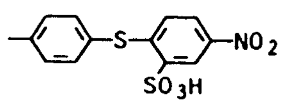 | rotbraun | | | | |
| 39 | 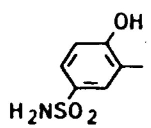 | 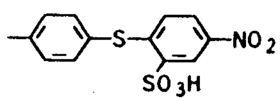 | | braun | | | gelbbraun |

Tabelle 3 (Forts.)

| Bsp. | A | R | Nuance der Färbung der Metallkomplexe auf Leder Cu | Cr | Co | Ni | Fe |
|---|---|---|---|---|---|---|---|
| 40 | 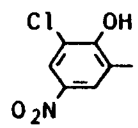 | 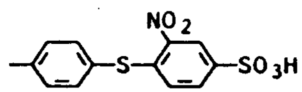 | | | rotbraun | | gelbbraun |
| 41 | 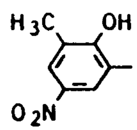 | 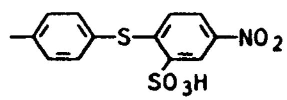 | rotbraun | | | rotbraun | |
| 42 | 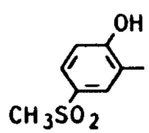 | 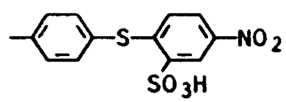 | | rotbraun | | | gelbbraun |
| 43 | 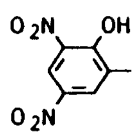 | 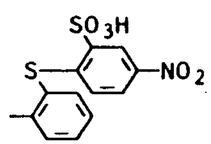 | | rotbraun | | | gelbbraun |

Tabelle 3 (Forts.)

| Bsp. | A | R | Nuance der Färbung der Metallkomplexe auf Leder: Cu | Cr | Co | Ni | Fe |
|---|---|---|---|---|---|---|---|
| 44 | 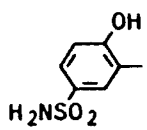 | 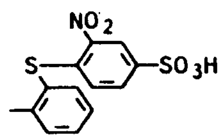 | rotbraun | | | rotbraun | gelbbraun |
| 45 | 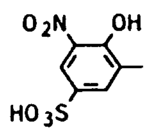 | 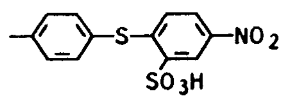 | rotbraun | | | | gelbbraun |
| 46 | 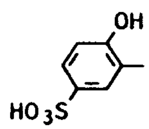 | 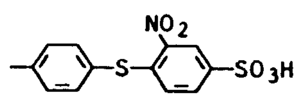 | | | rotbraun | | braun |

Beispiel 47

19,9 g 1-Amino-2-hydroxy-3,5-dinitrobenzol wurden in der üblichen Weise diazotiert und alkalisch auf 11 g Resorcin gekuppelt. Anschließend fügte man zu der Suspension bei pH 8-9 das Diazoniumsalz aus

32,6 g 4-Amino-4'-nitro-diphenylsulfid-2'-sulfonsäure hinzu. Nachdem die Bildung des Disazofarbstoffes beendet war, gab man die Diazoverbindung aus 21,8 g 1-Amino-4-nitrobenzol-2-sulfonsäure in der Weise hinzu, daß die Kupplung bei einem pH-Wert zwischen 6 und 7 erfolgte. Nach beendeter Kupplung wurde das Reaktionsgemisch mit Essigsäure auf pH 5 eingestellt und mit wasserfreiem Eisen-III-chlorid analog Beispiel 1 komplexiert.

Nach erfolgter Metallisierung wurde der Farbstoff durch Zugabe von Natriumchlorid abgeschieden, isoliert und getrocknet. Der Eisenkomplex der Verbindung der Formel

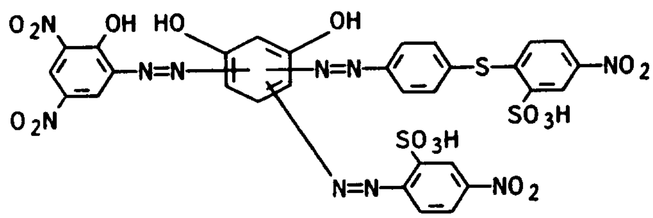

färbt vegetabilisch/synthetisch nachgegerbtes Chromrindleder in licht- und naßechten braunen Tönen.

Die in der folgenden Tabelle aufgeführten Farbstoffe werden in analoger Weise erhalten.

Tabelle 4

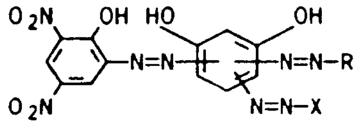

| Bsp. | R | X | Nuance des Fe-Komplexes auf Leder |
|---|---|---|---|
| 48 | 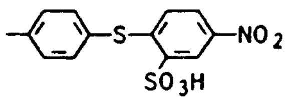 | 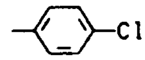 | gelbbraun |
| 49 | 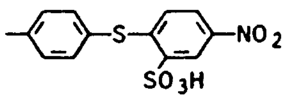 | 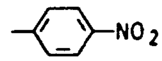 | braun |
| 50 | 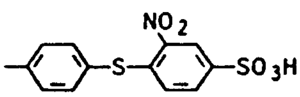 | 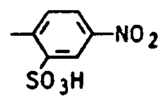 | gelbbraun |
| 51 | 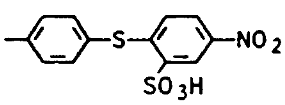 | 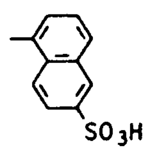 | braun |
| 52 | 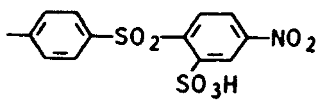 | 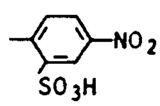 | braun |
| 53 | 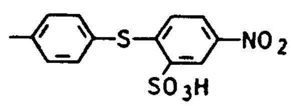 | 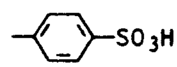 | braun |
| 54 | 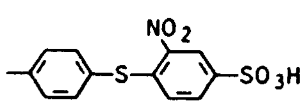 | 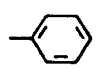 | braun |

Beispiel 55

In eine 90 °C heiße Schmelze von 39,5 g 4-Nitro-chlorbenzol in 250 ml Wasser wurden unter Rühren innerhalb etwa 15 Minuten 81 g 60 %iges Natriumsulfid, in 200 ml Wasser gelöst, zugetropft; dabei erwärmte sich das Reaktionsgemisch bis zum Sieden. Nach zwei Stunden bei unveränderter Temperatur wurde die Lösung von 4-Amino-thiophenol auf Raumtemperatur abgekühlt, mit 350 ml Wasser verdünnt und

der pH-Wert mit etwa 26 ml Essigsäure von 12 auf 8 zurückgestellt. Dann wurden 59,5 g 4-Nitrochlorbenzol-2-sulfonsäure zugefügt. Nach fünfstündigem Rühren bei 80 °C wurde die Lösung auf 60 °C abgekühlt und das entstandene Produkt mit verdünnter Salzsäure bei pH 1 ausgefällt und abgesaugt. Zur Reinigung wurde das Nutschgut bei 80 °C in 400 ml Wasser mit verdünnter Natronlauge bei pH 12 gelöst, filtriert, wie vorher beschrieben gefällt, abgesaugt und dann salzfrei und neutral gewaschen. Nach dem Trocknen bei 80 °C unter vermindertem Druck wurden 70 g 4-Amino-4'-nitro-diphenylsulfid-2'-sulfonsäure der Formel

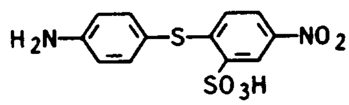

erhalten (entspr. ca. 86 % der Theorie, bezogen auf eingesetztes 4-Nitrochlorbenzol).

Die in der folgenden Tabelle 5 aufgeführten Anilinderivate werden in analoger Weise erhalten.

Tabelle 5

| Verb.Nr. | |
|---|---|
| 56 | 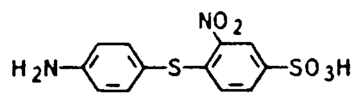 |
| 57 | 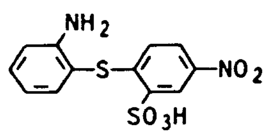 |
| 58 | 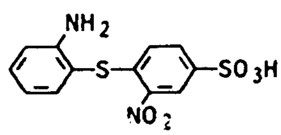 |
| 59 | 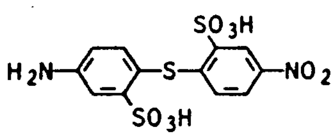 |
| 60 | 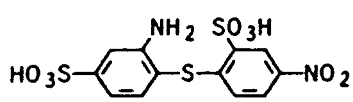 |
| 61 | 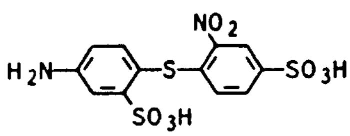 |

Tabelle 5 (Forts.)

| Verb.Nr. | |
|---|---|
| 62 | 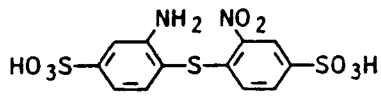 |
| 63 | 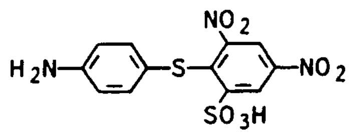 |
| 64 | 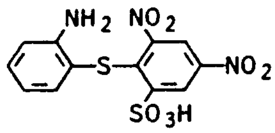 |

Beispiel 65 (nicht beansprucht)

55 g Thiophenol und 250 g 4-Nitro-chlorbenzol-2-sulfonsäure wurden in 1000 ml Wasser unter Rühren vorgelegt. Danach wurde der pH-Wert des Reaktionsgemisches mit verdünnter Natronlauge unter Wärmetönung bis 55°C auf 11 erhöht. Der Ansatz wurde dann zwei Stunden bei 50°C und danach weitere zwei Stunden bei 80°C gerührt, mit 100 g Natriumchlorid versetzt, auf Raumtemperatur abgekühlt und abgesaugt. Dann wurde der Filterkuchen bei 85 bis 90°C portionsweise in ein Reduktionsgemisch, erzeugt aus 200 g Eisenpulver, 600 ml Wasser und 150 ml 5n-Salzsäure, eingetragen. Die Reduktion war nach einer Stunde bei 90°C beendet. Nachdem der pH-Wert mit verdünnter Natronlauge auf 10 bis 11 erhöht worden war, wurde der Eisenschlamm heiß abfiltriert, mit heißem Wasser gut ausgewaschen und das Filtrat bei Raumtemperatur mit verdünnter Salzsäure mineralsauer gestellt. Der Niederschlag des Anilinderivats wurde abgesaugt und mit kaltem Wasser neutral gewaschen. Nach dem Trocknen unter vermindertem Druck bei 80°C wurden 121 g 4-Amino-diphenylsulfid-2-sulfonsäure der Formel

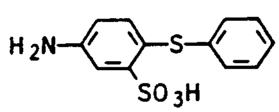

erhalten (entspr. ca. 84 % der Theorie, bezogen auf eingesetztes Thiophenol).

Beispiel 66 (nicht beansprucht)

32,6 g 4-Amino-4'-nitro-diphenylsulfid-2'-sulfonsäure wurden unter Rühren in 300 ml Wasser mit verdünnter Natronlauge bei pH 8 bis 9 gelöst, 15 g Soda zugegeben und bei 50°C 30 ml Essigsäureanhydrid zugetropft. Nachdem die Acetylierung beendet war, wurde die Lösung auf 90°C aufgeheizt und portionsweise 50 g Eisenpulver innerhalb von 30 Minuten eingetragen. Nach weiteren 30 Minuten im unveränderten Temperaturbereich war die Reduktion beendet, und es wurde analog dem Beispiel 81 weiterverfahren. Nach dem Trocknen wurden 23 g 4-Amino-4'-acetylamino-diphenylsulfid-2-sulfonsäureder Formel

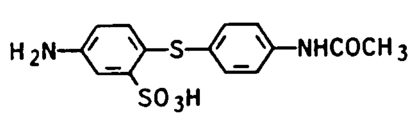

erhalten.

Beispiel 67 (nicht beansprucht)

32,6 g 4-Amino-4'-nitro-diphenylsulfid-2'-sulfonsäure wurden analog Beispiel 66 acetyliert und nach Verlauf der Reaktion bei 80 °C innerhalb einer Stunde mit 30 ml 30 %igem Wasserstoffperoxid versetzt. Nach 30 Minuten wurde das Oxidationsprodukt alkalisch bei Siedetemperatur verseift, heiß filtriert, das Filtrat mit konz. Salzsäure auf einen pH-Wert von 6 bis 7 gestellt und dann unter vermindertem Druck zur Trockene eingedampft. Der Rückstand wurde, um anorganische Salze abzutrennen, mit Methanol ausgekocht und das Filtrat wiederum unter vermindertem Druck eingedampft. Die zurückgebliebene rohe 4-Amino-4'-nitro-diphenylsulfon-2'-sulfonsäure (Na-Salz) der Formel

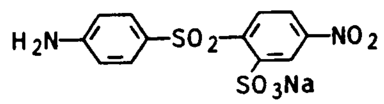

wurde in Aceton aufgenommen, verrührt, abgesaugt und getrocknet (Ausbeute: 33 g).

Beispiel 68 (nicht beansprucht)

730 ml Wasser und 159 g $Na_2SO_3$ (95 gew.%ig) wurden vorgelegt. Dann wurden bei 70 °C 530 g 4-Acetylaminobenzolsulfonsäurechlorid portionsweise unter Rühren eingetragen, gleichzeitig wurde mit 95 ml Natronlauge (50 gew.%ig) der pH-Wert bei ca. 9 gehalten. Nach 3 Stunden Nachrühren bei 70 °C wurde von ungelösten Anteilen abfiltriert, bei 30 °C 250 g Eis zugegeben und unter Rühren mit 375 ml Salzsäure (17 gew.%ig) bei pH 1,0 gefällt, wobei die Temperatur $\leqq$ 10 °C gehalten wurde. Die resultierende Suspension wurde nach 1,5 Stunden abgesaugt und mit 1,5 l Eiswasser gewaschen. Das Nutschgut wurde nun in 400 ml Eiswasser verrührt und bei einer Temperatur von ca. 15 °C mit 74 ml Natronlauge (30 gew.%ig) bei pH 9,0 gelöst. Es resultierten 782 ml einer klaren Lösung, die 185 g 4-Acetylaminobenzolsulfinsäure-Natriumsalz enthielt.

654 ml dieser Lösung (entsprechend 154,7 g 4-Acetylaminobenzolsulfinsäure-Natriumsalz) wurden vorgelegt und unter Rühren bei einem pH-Wert von 6,2 365 g 2-Nitrochlorbenzol-4-sulfonsäure (50 gew.%ig) zugegeben. Dann wurde mit einigen Tropfen Natronlauge auf pH 7,0 gestellt. Nach Zugabe von 9 g $CaCO_3$ und 0,35 g Kupferpulver wurde 20 Stunden bei 100 °C gerührt und danach heiß abfiltriert.

Die resultierende Lösung wurde mit 180 ml Salzsäure (17 gew.%ig) versetzt und 4 Stunden unter Rückfluß gekocht. Die erhaltene Suspension wurde dann bei Raumtemperatur abgesaugt, das Nutschgut mit 2 l Eiswasser gewaschen und bei 70 °C unter vermindertem Druck getrocknet.

Ausbeute: 250 g 4-Amino-2'-nitrodiphenyl-4'-sulfonsäure der Formel

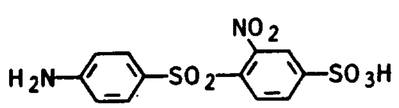

## Patentansprüche

1. Polyazofarbstoffe der Formel I

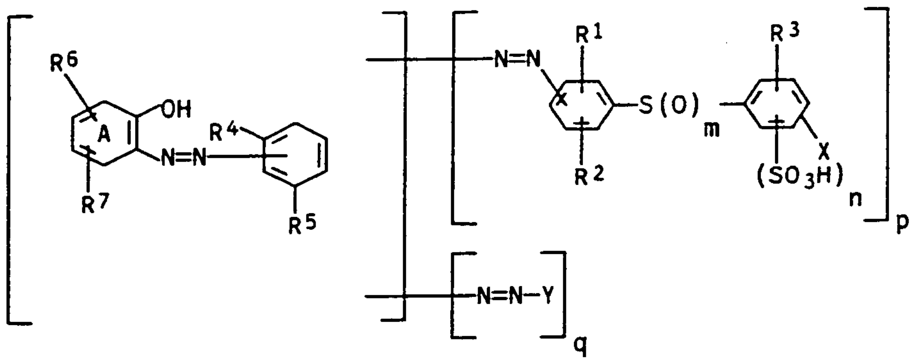

(I)

in freier Form oder als Kupfer-, Chrom-, Eisen-, Kobalt- oder Nickelkomplex, wobei

| | |
|---|---|
| m | 0, 1 oder 2 |
| n | 1, |
| p | 1 bis 2, |
| q | 0 bis 1, |
| X | Wasserstoff oder Nitro, |
| $R^1$ und $R^2$ | gleich oder verschieden sind und unabhängig voneinander jeweils Wasserstoff, $C_1$-$C_4$-Alkyl, $C_1$-$C_4$-Alkoxy, Halogen oder Hydroxysulfonyl, |
| $R^3$ | Wasserstoff, $C_1$-$C_4$-Alkoxy, Halogen, Carboxyl, Nitro oder $C_1$-$C_4$-Alkanoylamino, |
| $R^4$ und $R^5$ | gleich oder verschieden sind und unabhängig voneinander jeweils Hydroxy oder Amino, |
| $R^6$ | Wasserstoff, $C_1$-$C_4$-Alkyl, Halogen, Nitro, Hydroxysulfonyl, Sulfamoyl, $C_1$-$C_4$-Mono- oder -Dialkylsulfamoyl oder $C_1$-$C_4$-Alkylsulfonyl, |
| $R^7$ | Wasserstoff, Halogen, Nitro oder Hydroxysulfonyl und |
| Y | einen Rest der Formel |

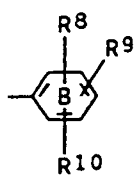

oder

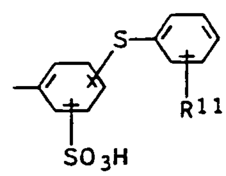

worin

| | |
|---|---|
| $R^8$ | für Wasserstoff, Hydroxy, Halogen, Nitro oder Hydroxysulfonyl, |
| $R^9$ | für Wasserstoff, Halogen, Nitro oder Hydroxysulfonyl, |
| $R^{10}$ | für Wasserstoff, $C_1$-$C_4$-Alkyl, $C_1$-$C_4$-Alkoxy oder Hydroxysulfonyl und |
| $R^{11}$ | für Wasserstoff, $C_1$-$C_4$-Alkyl, $C_1$-$C_4$-Alkoxy, Halogen oder Carboxyl stehen, bedeuten und die |

Ringe A und B jeweils durch einen gegebenenfalls durch Nitro oder Hydroxysulfonyl substituierten Benzoring anelliert sein können.

2. Polyazofarbstoffe gemäß Anspruch 1, die der Formel Ia entsprechen

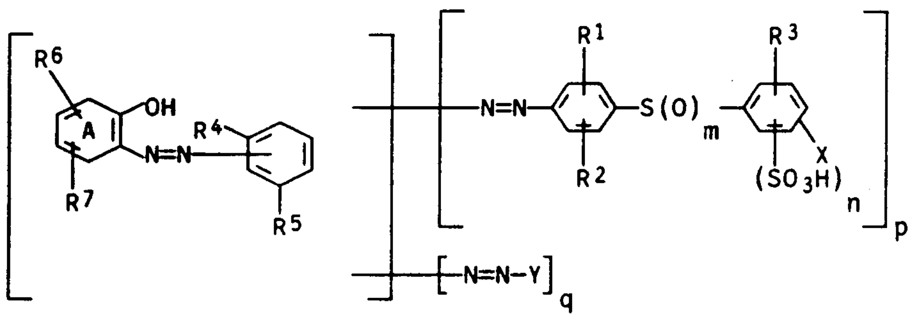

(Ia),

in der $R^3$ Wasserstoff bedeutet und $R^1$, $R^2$, $R^4$, $R^5$, $R^6$, $R^7$, X, Y, m, n, p, q sowie der Ring A jeweils die in Anspruch 1 genannte Bedeutung besitzen.

3. Polyazofarbstoffe gemäß Anspruch 1, dadurch gekennzeichnet, daß X Nitro, m 0, q 0, $R^1$ und $R^2$ jeweils Wasserstoff und $R^3$ Wasserstoff oder Nitro bedeuten.

4. Anilinderivate der Formel II

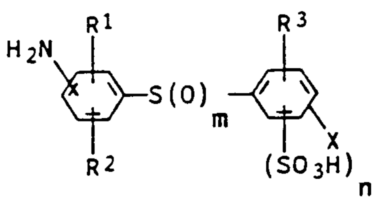

(II),

in der

| | |
|---|---|
| m | 0, |
| n | 1, |
| X | Wasserstoff oder Nitro, |
| $R^1$ und $R^2$ | gleich oder verschieden sind und unabhängig voneinander jeweils Wasserstoff, $C_1$-$C_4$-Alkyl, $C_1$-$C_4$-Alkoxy oder Hydroxysulfonyl, und |
| $R^3$ | Wasserstoff, $C_1$-$C_4$-Alkoxy, Carboxyl, Nitro oder $C_1$-$C_4$-Alkanoylamino bedeuten. |

## Claims

1. A polyazo dye of the formula I where

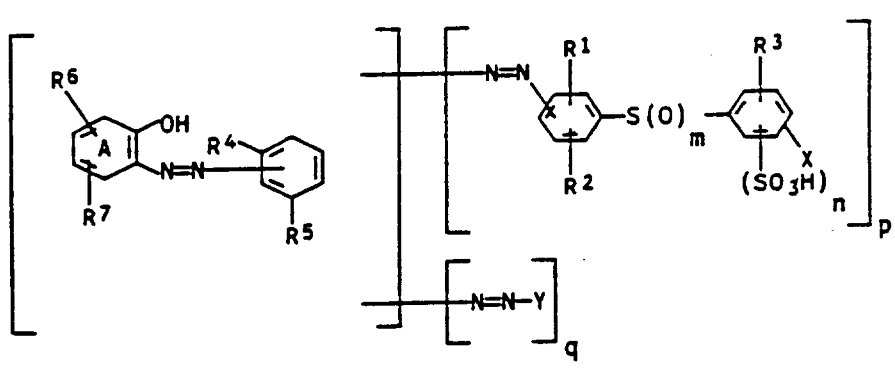

(I)

| | |
|---|---|
| m | is 0, 1 or 2, |

n is 1,
p is from 1 to 2,
q is from 0 to 1,
x is hydrogen or nitro,
$R^1$ and $R^2$ are identical or different and each is independently of the other hydrogen, $C_1$-$C_4$-alkyl, $C_1$-$C_4$-alkoxy, halogen or hydroxysulfonyl,
$R^3$ is hydrogen, $C_1$-$C_4$-alkoxy, halogen, carboxyl, nitro or $C_1$-$C_4$-alkanoylamino,
$R^4$ and $R^5$ are identical or different and each is independently of the other hydroxyl or amino,
$R^6$ is hydrogen, $C_1$-$C_4$-alkyl, halogen, nitro, hydroxysulfonyl, sulfamoyl, $C_1$-$C_4$-monoalkylsulfamoyl, $C_1$-$C_4$-dialkylsulfamoyl or $C_1$-$C_4$-alkylsulfonyl,
$R^7$ is hydrogen, halogen, nitro or hydroxysulfonyl and
Y is a radical of the formula

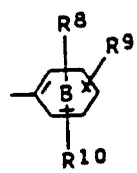

or

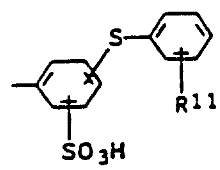

where
$R^8$ is hydrogen, hydroxyl, halogen, nitro or hydroxysulfonyl,
$R^9$ is hydrogen, halogen, nitro or hydroxysulfonyl,
$R^{10}$ is hydrogen, $C_1$-$C_4$-alkyl, $C_1$-$C_4$-alkoxy or hydroxysulfonyl and
$R^{11}$ is hydrogen, $C_1$-$C_4$-alkyl, $C_1$-$C_4$-alkoxy, halogen or carboxyl, and the

rings A and B may each be fused with an unsubstituted or nitro- or hydroxysulfonyl-substituted benzo ring, in the free form or as a copper, chromium, iron, cobalt or nickel complex.

2. A polyazo dye as claimed in claim 1, of the formula Ia

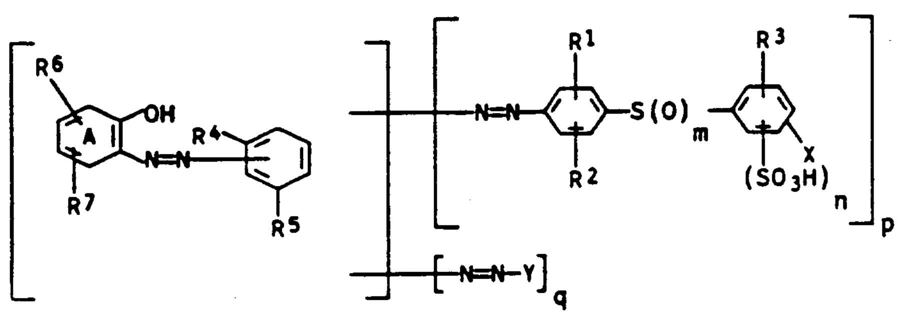

(Ia),

where $R^3$ is hydrogen and $R^1$, $R^2$, $R^4$, $R^5$, $R^6$, $R^7$, X, Y, m, n, p, q and the ring A are each as defined in claim 1.

3. A polyazo dye as claimed in claim 1, wherein X is nitro, m is 0, q is 0, $R^1$ and $R^2$ are each hydrogen, and $R^3$ is hydrogen or nitro.

4. An aniline derivative of the formula II

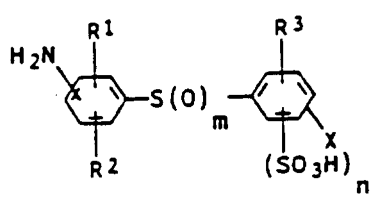

(II),

where

m is 0,

n is 1,

X is hydrogen or nitro,

$R^1$ and $R^2$ are identical or different and each is independently of the other hydrogen, $C_1$-$C_4$-alkyl, $C_1$-$C_4$-alkoxy or hydroxysulfonyl, and

$R^3$ is hydrogen, $C_1$-$C_4$-alkoxy, carboxyl, nitro or $C_1$-$C_4$-alkanoylamino.

## Revendications

**1.** Colorants polyazoïques de formule I

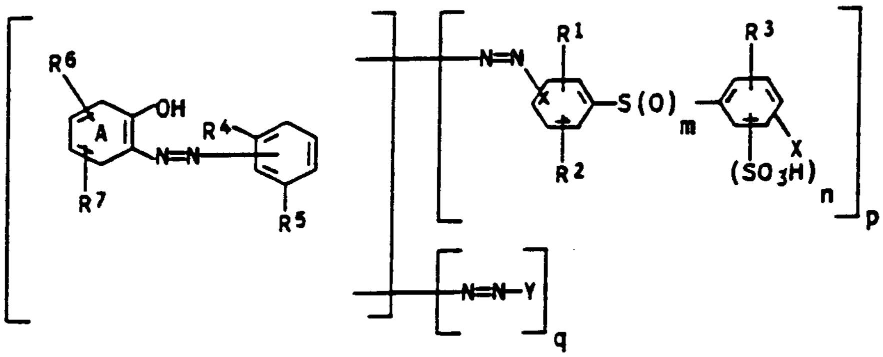

(I)

sous forme libre ou sous forme de complexe avec du cuivre, du chrome, du fer, du cobalt ou du nickel,

m étant mis pour 0, 1 ou 2,

n étant mis pour 1,

p étant mis pour 1 à 2

q étant mis pour 0 à 1,

X représentant un atome d'hydrogène ou un groupement nitro,

$R^1$ et $R^2$ étant identiques ou différents et représentant chacun, indépendamment l'un de l'autre, un atome d'hydrogène, un reste alkyle en $C_1$-$C_4$, alcoxy en $C_1$-$C_4$, un atome d'halogène ou un groupement hydroxysulfonyle,

$R^3$ représentant un atome d'hydrogène, un reste alcoxy en $C_1$-$C_4$, un atome d'halogène ou un groupement carboxyle, nitro ou alcanoylamino en $C_1$-$C_4$,

$R^4$ et $R^5$ étant identiques ou différents et représentant chacun, indépendamment l'un de l'autre, un groupement hydroxy ou amino,

$R^6$ représentant un atome d'hydrogène, un reste alkyle en $C_1$-$C_4$, un atome d'halogène ou un groupement nitro, hydroxysulfonyle, sulfamoyle, mono- ou dialkylsulfamoyle en $C_1$-$C_4$ ou alkylsulfonyle en $C_1$-$C_4$,

$R^7$ représentant un atome d'hydrogène, d'halogène ou un groupement nitro ou hydroxysulfonyle et

Y représentant un reste de formule

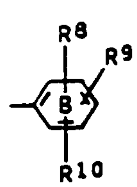

ou

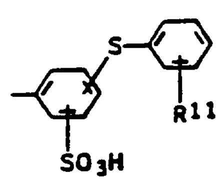

dans laquelle

$R^8$ est mis pour un atome d'hydrogène, pour un radical hydroxy, pour un atome d'halogène ou pour un groupement nitro ou hydroxysulfonyle,

$R^9$ est mis pour un atome d'hydrogène, d'halogène ou pour un groupement nitro ou

hydroxysulfonyle,

$R^{10}$ est mis pour un atome d'hydrogène ou pour un reste alkyle en $C_1$-$C_4$, alcoxy en $C_1$-$C_4$ ou hydroxysulfonyle et

$R^{11}$ est mis pour un atome d'hydrogène, pour un reste alkyle en $C_1$-$C_4$, alcoxy en $C_1$-$C_4$, pour un atome d'halogène ou pour un groupement carboxyle,

les noyaux A et B pouvant être condensés chacun par un noyau benzénique éventuellement substitué par un groupement nitro ou hydroxysulfonyle.

**2.** Colorants polyazoïques selon la revendication 1 qui répondent à la formule Ia

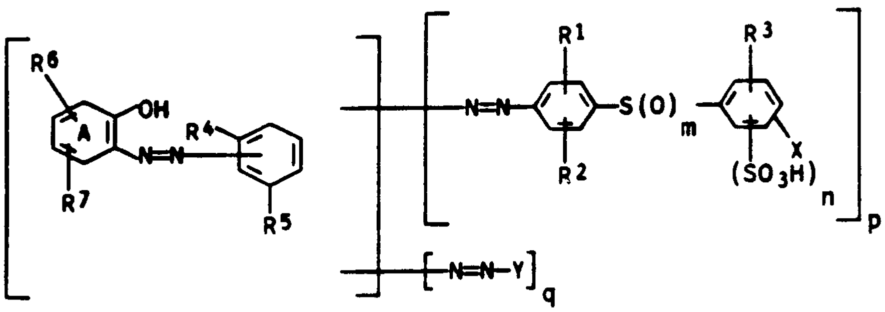

(Ia),

dans laquelle $R^3$ représente un atome d'hydrogène et $R^1$, $R^2$, $R^4$, $R^5$, $R^6$, $R^7$, X, Y, m, n, p, q ainsi que le noyau A ont chacun la signification indiquée dans la revendication 1.

**3.** Colorants polyazoïques selon la revendication 1, caractérisés en ce que X représente un groupement nitro, m est mis pour 0, q pour 0, $R^1$ et $R^2$ représentent chacun un atome d'hydrogène et $R^3$ représente un atome d'hydrogène ou un groupement nitro.

**4.** Dérivés d'aniline de formule II

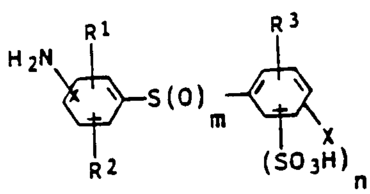

(II),

dans laquelle

m est mis pour 0.

n est mis pour 1,

X représente un atome d'hydrogène ou un groupement nitro,

$R^1$ et $R^2$ sont identiques au différents et représentent chacun, indépendamment l'un de l'autre, un atome d'hydrogène, un reste alkyle en $C_1$-$C_4$, alcoxy en $C_1$-$C_4$ ou hydroxysulfonyle, et

$R^3$ représente un atome d'hydrogène ou un reste alcoxy en $C_1$-$C_4$, carboxyle, nitro ou alcanoylamino en $C_1$-$C_4$.